# EUROPEAN PATENT APPLICATION

(11) **EP 3 889 573 A1**
(43) Date of publication of application: **06.10.2021**
(21) Application number: 19888962.8
(22) Date of filing: 26.11.2019
(51) Int. Cl.: G01N 1/30, G01N 33/576, G01N 33/68

(54) **SAMPLE PRETREATMENT METHOD FOR VIRUS DETECTION**

(30) Priority: 30.11.2018 KR 20180152642
(71) Applicant: Lee, Hong Jai, Seoul 05501 (KR)
(72) Inventor: Lee, Hong Jai, Seoul 05501 (KR)
(74) Representative: Schnappauf, Georg
(86) International application number: PCT/KR2019/016321
(87) International publication number: WO 2020/111708

(57) **Abstract**

The present invention relates to a method for detecting a virus, and more particularly, to a method for detecting a virus including a lipid bilayer envelope, wherein the method allows a surface antigen, which is a lipid bilayer envelope protein, to be effectively exposed from lipoproteins that mask all or part of the envelope protein, thereby revealing the surface antigen that is masked by the lipoproteins, so that a probe such as an antibody can be used to detect the virus with high sensitivity within a short time.

## Description

### Technical Field

The present invention relates to a method for pretreating a sample for virus detection, and more particularly, to a method for pretreating a biological sample, by which the envelope protein HCeAg E1 or E2 of hepatitis C virus (HCV), which is an enveloped virus, can be exposed for detection of an HCV surface antigen, and a method for detecting an HCV envelope antigen using the same.

### Background Art

In general, virion, which is a viral particle that has the ability to infect host cells, includes a core genome containing genetic material such as DNA or RNA, and a capsid, which is a protein shell that functions to protect such genetic material, and further includes an envelope in a case of an enveloped virus. Types of the enveloped viruses include DNA viruses such as herpesvirus; RNA viruses such as flavivirus and retrovirus; and the like.

Viruses (hepatitis viruses) that cause hepatitis may be classified into enveloped viruses (types B, C, and D) and non-enveloped viruses (types A and E). Among the enveloped viruses, hepatitis B virus (hereinafter referred to as 'HBV'), which is estimated to have an infection rate of about 350 million people worldwide, is a major cause of chronic liver disease. The HBV is a virus having a DNA genome which belongs to the hepadnavirus family and causes acute and chronic hepatitis. In particular, the prevalence of HBV, which was such that a percentage of chronically infected patients reached about 5% to 8% in Korea and China, has been decreased to some extent due to the development of vaccines. However, the truth is that a large number of chronic hepatitis patients still exist due to continuing outbreak of the mother-to-child vertical HBV infection. Chronic HBV infection causes liver cancer as well as hepatitis and cirrhosis. The WHO's research has demonstrated that about 80% of liver cancers is caused by chronic hepatitis B, and the incidence of liver cancer is about 300 times higher in infected people than non-infected people.

On the other hand, in a case of hepatitis C virus (hereinafter referred to as 'HCV'), a method capable of directly detecting HCV viral particles present in the blood has not been developed, and diagnosis thereof is currently only made with HCV RNA and HCV antibodies. Since HCV RNA itself is unstable, there may be severe deviations in determination of the exact amount thereof due to factors such as transport process, temperature, and time taken for testing. In addition, a processing method for RT-PCR, which is currently used for human specimens, has a limitation in that for a specimen of different species, it is not possible to detect HCV RNA therein. For HCV antibody tests that are commonly used, since antibodies are not produced immediately after infection, it is not possible to detect HCV during a non-antibody-production period, which is before antibodies appear in the blood. In addition, various drugs such as interferon and ribavirin, which are involved in treatment of hepatitis C patients, may be very useful; however, it is not enough to measure an amount of antibodies for selective application of such a treatment method and observation of treatment progress.

The methods for detecting HCV gene include nucleic acid amplification tests (NAT) and DNA probe methods, which are currently widely used in clinical settings. The nucleic acid amplification test has high sensitivity, whereas this test is disadvantageous in that loss, contamination, or the like of RNA may occur in reverse transcription process for performing polymerase chain reaction (PCR), and special amplification equipment is required. Furthermore, the DNA probe method has a limitation in that the method has low detection sensitivity; it takes about 20 hours to obtain a result; and a level of HCV RNA present in the blood of an HCV-infected person is only 10² to 10⁷ IU/ml.

To overcome such a limitation, methods for detecting E1 or E2 protein, which is an HCV surface antigen, wherein screening can be performed simply, quickly, and in large quantities, are attracting attention. Currently, however, no method has been developed which is capable of conveniently checking presence or absence of HCV in clinical practice. Moreover, there is still a limitation in that an HCV detection method should be selected depending on presence or absence of HCV antibodies in the patient's blood; and in the absence of HCV antibodies, HCV RNA must be detected by RT-PCR that is time-consuming and expensive.

### Technical Problem

An object of the present invention is to provide a method for pretreating a sample for detection of an envelope protein of a lipid bilayer envelope-containing virus.

Another object of the present invention is to provide a composition for pretreating a lipid bilayer envelope-containing virus for detection of an envelope protein of the virus.

Yet another object of the present invention is to provide a method for detecting an enveloped virus.

However, the technical problem to be solved by the present invention is not limited to the above-mentioned problems, and other problems which are not mentioned will be clearly understood by those skilled in the art from the following description.

### Solution to Problem

The present inventors have found that for HCV surface E antigen (hereinafter referred to as 'HCeAg'), which is in a state where the antigen is masked by lipoproteins such as low-density lipoprotein cholesterol (LDL-cholesterol) in human blood and is therefore difficult to detect, pretreatment with a bile acid or a bile acid derivative at a high concentration, or pretreatment with weak acidity causes the HCeAg, which is masked by lipoproteins and is therefore difficult to detect, to be exposed, so that detection of the HCeAg can be very efficiently achieved; and thus have completed the present invention.

In an embodiment of the present invention, there is provided a method for pretreating a sample for virus detection.

The pretreatment method of the present invention comprises steps of: isolating an enveloped virus including a lipid bilayer envelope, from a biological sample isolated from a target individual; and treating the enveloped virus with any one or more selected from the group consisting of a bile acid, a bile acid derivative, and a pH adjusting agent.

In the present invention, the enveloped virus including a lipid bilayer envelope may be an enveloped virus that includes a core genome containing genetic material such as DNA or RNA, and a capsid, which is a protein shell that functions to protect such genetic material; and an envelope containing a lipoprotein such as a lipid bilayer.

In the present invention, the virus may include any virus as long as the virus further includes an envelope containing a lipid bilayer in addition to a capsid constituting the virus itself, and examples thereof include, but are not limited to, hepadnavirus, poxvirus, herpesvirus, and the like, which have DNA as genetic material; and coronavirus, filovirus, rhabdovirus, arenavirus, flavivirus, retrovirus, and the like, which have RNA as genetic material. Preferably, the virus may be a hepatitis virus, with hepatitis C virus (HCV) being more preferred. However, the virus is not limited thereto.

In the present invention, the treating step may be carried out such that an envelope protein is exposed from the lipid bilayer. The enveloped virus, in particular, HCV, is present in the blood of patients with hepatitis in a form in which its genomic RNA is packed by a core protein, and an envelope protein, for example, HCeAg of the enveloped virus, such as surface antigen E1 or E2, is anchored to a lipid bilayer surrounding the core protein, and in which part or all of HCeAg is masked by or combined with lipoproteins present in the blood. Therefore, for the purpose of the present invention, there is an advantage in that in a case where acidity of a sample is adjusted using the bile acid, the bile acid derivative, or the pH adjusting agent, HCeAg, which is difficult to detect due to lipoproteins, is externally exposed through a single step, so that the sample is in a state suitable for detection using a probe or antibody.

In the present invention, for humans, the bile acid is a primary bile acid synthesized from cholesterol in the liver, which is divided into cholic acid and chenodeoxycholic acid; and a second bile acid is produced from the primary bile acid through metabolism by intestinal bacteria in the large intestine, in which cholic acid is metabolized to deoxycholic acid or chenodeoxycholic acid to lithocholic acid. In addition, the bile acid may be conjugated with glycine or taurine in the liver, and thus converted into taurocholic acid and glycocholic acid (which are derivatives of cholic acid), taurochenodeoxycholic acid and glycochenodeoxycholic acid (which are derivatives of chenodeoxycholic acid), glycodeoxycholic acid and taurodeoxycholic acid (which are derivatives of deoxycholic acid), or glycolithocholic acid and taurolithocholic acid (which are derivatives of lithocholic acid). On the other hand, for rodents, muricholic acid is present as a secondary bile acid.

In the present invention, the bile acid or the bile acid derivative may be any one or more selected from the group consisting of cholic acid, chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid, with taurochenodeoxycholic acid being preferred. However, the present invention is not limited thereto.

In the present invention, in a case of treating the virus, the bile acid or the bile acid derivative may be used at a concentration of 10 to 100 µmol/L, preferably 50 to 100 µmol/L, and more preferably 80 to 100 µmol/L. However, the present invention is not limited thereto. In a case where the bile acid is used at a concentration of lower than 10 µmol/L, the envelope protein cannot be sufficiently exposed from lipoproteins that mask the viral envelope; and in a case where the bile acid is used at a concentration of higher than 100 µmol/L, the viral envelope-containing particle structure's stability and the like may be impaired.

In the present invention, the pH adjusting agent may be an agent which adjusts pH in the HCV-containing serum so that the bile acid or the bile acid derivative can expose an envelope protein from lipoproteins masking the envelope, and examples thereof include hydrochloric acid, sulfuric acid, acetic acid, trifluoroacetic acid, trichloroacetic acid, ammonium hydroxide, potassium hydroxide, ammonia, and the like, with hydrochloric acid being preferred. However, the pH adjusting agent is not limited thereto.

The pH adjusting agent may be used to adjust the pH of a culture environment for the HCV to pH 4 to pH 7. Preferably, the pH may be pH 5 to pH 7. In a case where the pH of the culture environment for the HCV is lower than pH 4 or higher than pH 7, surface proteins of an enveloped virus, in particular, HCV, composed of proteins may be denatured, thereby rather leading to decreased detection efficiency.

In the present invention, the target individual may be an individual who is infected with or suspected of being infected with an enveloped virus, in particular HCV, and may include both humans (normal persons or patients) and animals (experimental animals and the like).

In the present invention, the biological sample may be, but is not limited to, whole blood, serum, plasma, saliva, urine, sputum, lymph fluid, cells, or the like, isolated from a target individual.

The pretreatment method of the present invention may be performed simultaneously with or before performing a conventional method for detecting the enveloped virus including a lipid bilayer envelope.

In another embodiment of the present invention, there is provided a composition for pretreating a sample for virus detection.

The composition of the present invention comprises, as an active ingredient, any one or more selected from the group consisting of a bile acid, a bile acid derivative, and a pH adjusting agent.

In the present invention, the composition allows a lipid bilayer, in particular, an envelope protein, which is partially or entirely masked by lipoproteins, to be exposed, thereby leading to remarkably increased detection efficiency of the enveloped virus, in which the envelope protein is, in particular, HCeAg that is an HCV envelope protein.

In addition to the bile acid or the bile acid derivative, the composition of the present invention may further comprise a substance capable of exposing HCeAg, which is partially or entirely masked by lipoproteins, in which the substance may be, for example, a surfactant or an acidifying agent.

In the present invention, the enveloped virus including a lipid bilayer envelope, which is detected using the composition, can be used in the above-described pretreatment method, and thus description of the details related to the enveloped virus including a lipid bilayer envelope, the bile acid, the bile acid derivative, the pH adjusting agent, and the like is omitted to avoid excessive complexity of the specification due to repeated description.

In yet another embodiment of the present invention, there is provided a method for detecting an enveloped virus.

The method for detecting an enveloped virus of the present invention comprises steps of: isolating an enveloped virus including a lipid bilayer envelope, from a biological sample isolated from a target individual; treating the enveloped virus with any one or more selected from the group consisting of a bile acid, a bile acid derivative, and a pH adjusting agent; and adding thereto an antibody specific for the virus.

The method for detecting an enveloped virus of the present invention may be achieved by further performing a conventional method for detecting a virus, for example, a step of reacting a virus with an antibody specific for an antigen of the virus, after performing the pretreatment method for detecting an enveloped virus of the present invention. In the method for detecting an enveloped virus, it may be determined that the enveloped virus is present in the biological sample in a case where the antibody specific for the virus has caused an antigen-antibody reaction.

In the present invention, the antibody may be specific for an envelope protein, such as E1 or E2 protein that is a surface antigen, of the virus. However, the present invention is not limited thereto.

In the present invention, the conventional method for detecting a virus may be, but is not limited to, any one or more methods selected from the group consisting of immunohistochemical staining, enzyme-linked immunosorbent assay (ELISA), radioimmunoassay (RIA), enzyme immunoassay (EIA), fluorescence immunoassay (FIA), and luminescence immunoassay (LIA).

In the present invention, the enveloped virus including a lipid bilayer envelope, which is used in the method for detecting an enveloped virus, can be used in the above-described pretreatment method, and thus description of the details related to the enveloped virus including a lipid bilayer envelope, the bile acid, the bile acid derivative, the pH adjusting agent, the biological sample, the envelope protein, the exposure of the envelope protein, the target individual, and the like is omitted to avoid excessive complexity of the specification due to repeated description.

### Advantageous Effects of Invention

The method for pretreating a sample for virus detection of the present invention allows a surface antigen, which is a lipid bilayer envelope protein, to be effectively exposed from lipoproteins that mask all or part of the envelope protein, thereby revealing the surface antigen that is masked by the lipoproteins, so that a probe such as an antibody can be used to detect the virus with high sensitivity within a short time.

### Brief Description of Drawings

FIG. 1 illustrates results for stability of E2 surface antigen and genome of HCV, depending on HCV host and non-host humoral milieu, according to an embodiment of the present invention.
FIG. 2 illustrates results for destruction of HCV, that is, detection of HCV core antigen exposed from lipoproteins, depending on HCV host and non-host humoral milieu, according to an embodiment of the present invention.
FIGS. 3A to 3D illustrate results for interaction with lipids in the presence or absence of HCV, depending on HCV host and non-host humoral milieu, according to an embodiment of the present invention.
FIG. 4 illustrates results obtained by identifying detection levels of HCV RNA, depending on concentrations of a bile acid derivative, according to an embodiment of the present invention.
FIG. 5 illustrates results obtained by identifying detection levels of HCV E2 surface antigen, depending on concentrations of a bile acid derivative, according to an embodiment of the present invention.
FIGS. 6A and 6B illustrate results obtained by identifying detection levels of HCV RNA and E2 surface antigen, depending on pH changes in a culture environment for HCV, according to an embodiment of the present invention.

### Detailed Description of Invention

In an embodiment of the present invention, there is provided a method for pretreating a sample for virus detection, comprising steps of: isolating an enveloped virus including a lipid bilayer envelope, from a biological sample isolated from a target individual; and treating the enveloped virus with any one or more selected from the group consisting of a bile acid, a bile acid derivative, and a pH adjusting agent.

In another embodiment of the present invention, there is provided a composition for pretreating a sample for enveloped virus detection, comprising, as an active ingredient, any one or more selected from the group consisting of a bile acid, a bile acid derivative, and a pH adjusting agent.

Hereinafter, the present invention will be described in more detail by way of examples. These examples are only for describing the present invention in more detail, and it will be apparent to those skilled in the art that according to the gist of the present invention, the scope of the present invention is not limited by these examples.

### Examples

### [Preparation example] Isolation of enveloped virus

Normal serum (hepatitis B virus (HBV)- and hepatitis C virus (HCV)-negative) and chronic hepatitis C patient's serum (HCV-positive) were received from Wonju Severance Christian Hospital (Ethics Committee Approval No. CR316312). In addition, mouse (Balb c) serum (cat no. IMSBC-SER) was purchased from Innovative Research.

Each of the above-mentioned sera was added to a tube having 3 ml of a cushion buffer that contains 20 g/L of sucrose, 50 mmol/L of Tris-HCl (pH 7.5), and 30 mmol/L of NaCl. The mixture was centrifuged for 1 hour at 220,000 g. Then, the supernatant was removed from the tube, and 5 ml of phosphate-buffered saline (PBS) solution was added to the tube for dilution. Then, the HCV RNA contained in the dilution was measured through real-time PCR using the Roche's COBAS TaqMan system, to determine a concentration of the isolated virus.

### [Example 1] Identification of stability of E2 surface antigen and genome of HCV

It was identified how non-host humoral milieu affects genome stability and surface antigenicity of HCV.

The HCV isolated in the preparation example was added to a serum isolated from human blood, a serum isolated from mouse blood, and Dulbecco's Modified Eagle's Medium (DMEM), which is a cell culture medium, and culture was performed for 24 hours. Then, a concentration of the HCV RNA contained in each culture medium was measured through real-time PCR using the Roche's COBAS TaqMan system.

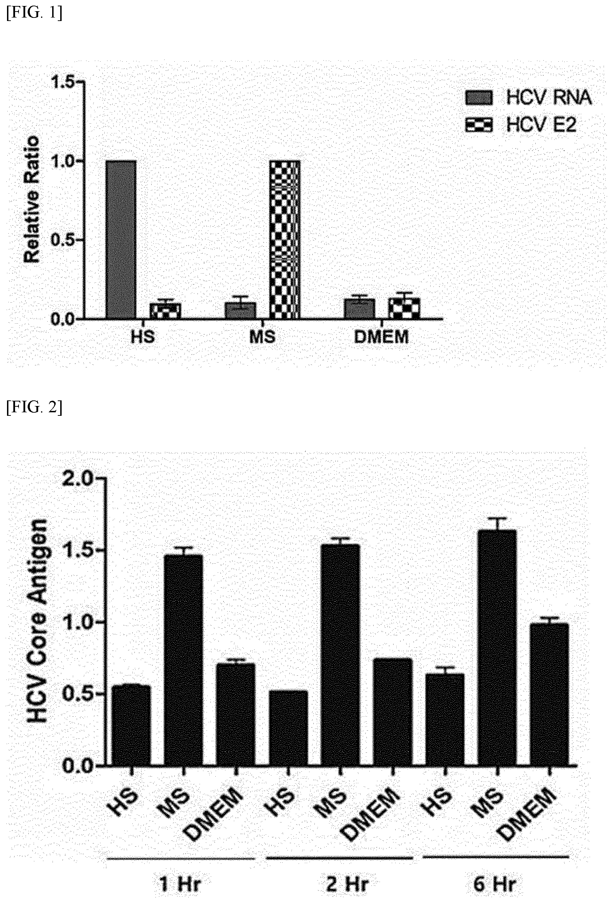

In addition, 100 µl/well of HCV anti-E2 antibody (Santa Cruz Biotechnology, Inc., cat no. sc-57769, USA) was added to a micro ELISA plate, and the reaction was allowed to proceed at 4°C overnight. Then, 200 µl/well of 1X ELISA/ELISPOT Diluent (Invitrogen, USA) was additionally added thereto, and the reaction was allowed to proceed for 1 hour. Each of the HCV-contained culture media was dispensed into each well, in which the reaction had been completed, and the reaction was allowed to proceed for 2 hours. Then, the HCV anti-E2 antibody diluted in a ratio of 1:30 with 100 µl of 1X ELISA/ELISPOT Diluent was added to the plate, and the reaction was allowed to proceed for 1 hour. Then, the additional reaction with 100 µl of HRP-conjugated anti-rabbit IgG was allowed to proceed for 30 minutes. Then, 100 µl of TMB solution (Invitrogen, USA) was added to each well, and absorbance was measured through a microreader at 450 nm. The results are illustrated in FIG. 1.

As illustrated in FIG. 1, the HCV RNA was present at a high level in the human serum (HS), but was present at a very low level in the mouse (MS) serum and the DMEM. The HCV E2 antigen was present at a low level in the human serum, but was present at a very high level in the mouse serum.

From the above results, it can be seen that the HCV RNA is present at a remarkably high level (that is, there are numerous viral particles) in the human (host) serum as compared with the non-host-derived serum, whereas the HCV E2 is present at a higher level in the mouse (non-host) serum, suggesting that the HCV's envelope is masked by lipoproteins or the like in the human (host) serum. On the other hand, it can be seen that due to the fact that humans and mice have different bile acid composition ratios, that is, mice have a higher proportion of dihydroxy bile acid (which is a more water-soluble bile acid), a degree of exposure of the envelope protein is affected by the types of bile acid.

### [Example 2] Identification of HCV core in non-host humoral milieu

To find out if the reason why HCV RNA remarkably decreases and E2 increases in mouse (non-host of HCV) serum is destruction of HCV, it was identified whether or not HCV core protein was detected.

In the same manner as in Example 1, the HCV isolated in the preparation example was added to a serum isolated from human blood, a serum isolated from mouse blood, and Dulbecco's Modified Eagle's Medium (DMEM), which is a cell culture medium, and culture was performed for 1 hour, 2 hours, or 6 hours. Then, it was identified whether or not HCV core was detected, using the same ELISA method as described for detection of E2 antigen in Example 1. The results are illustrated in FIG. 2. Here, the antibody used for ELISA was an anti-HCV core antigen antibody (Abcam, cat no. ab50288).

As illustrated in FIG. 2, it was identified that in a case where the HCV was cultured in the mouse serum, HCV core was detected at a higher level in the mouse (non-host) serum than in the host at 1 hour, and a degree of detection of HCV core increased, over time, in the mouse (non-host) serum and DMEM, which is a cell culture medium, as compared with the human (host) serum.

From the above results, it can be seen that in view of the fact that the core surrounded by an envelope is exposed in the non-host environment, HCV viral particles are destroyed and this can lead to increased detection of the envelope.

### [Example 3] Identification of interaction of HCV with lipids

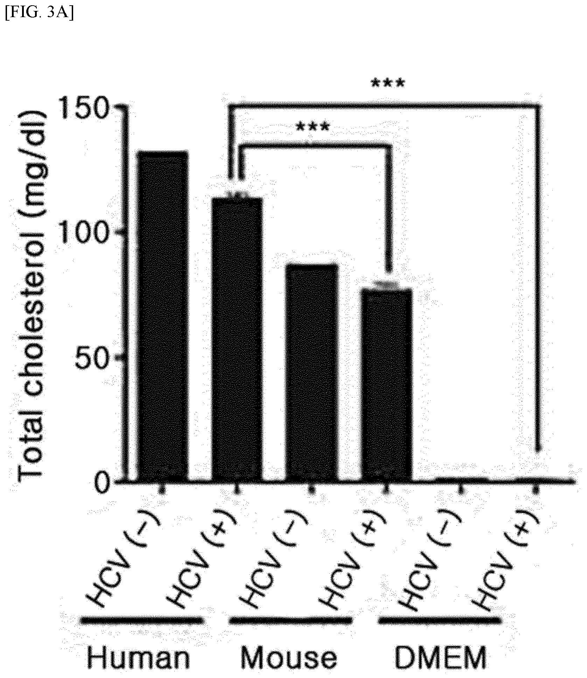

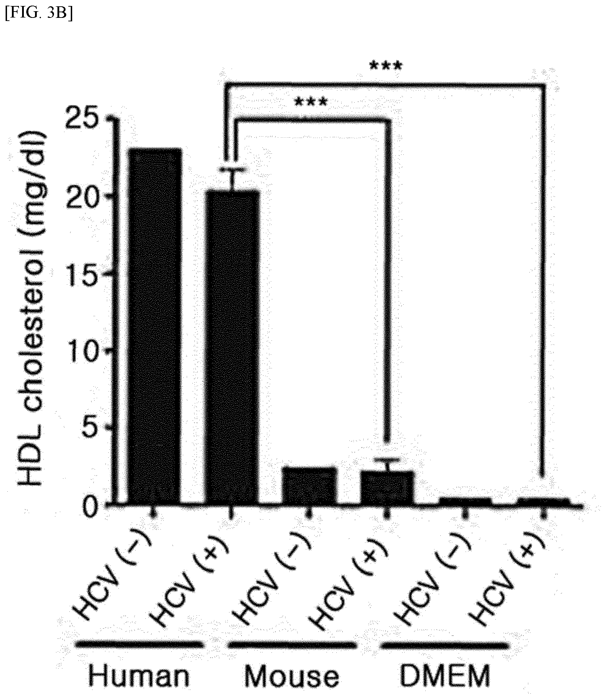

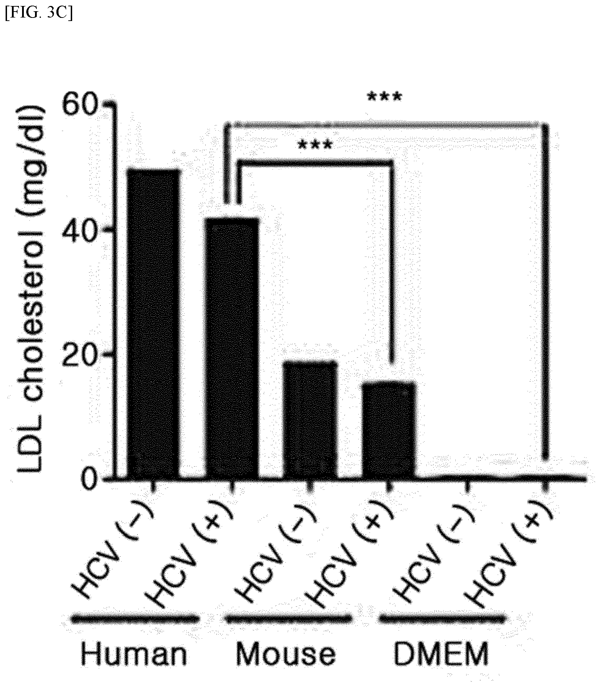

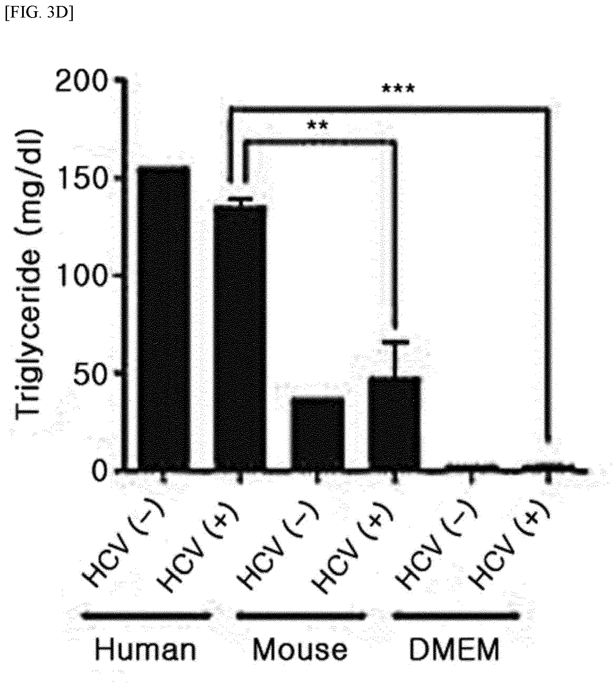

In the same manner as in Example 1, the HCV isolated in the preparation example was added to a serum isolated from human blood, a serum isolated from mouse blood, and Dulbecco's Modified Eagle's Medium (DMEM), which is a cell culture medium, and culture was performed for 24 hours. Then, amounts of lipids present in each culture medium were measured using ADVIA 1800 (Simens), and the results are illustrated in FIGS. 3A to 3D.

As illustrated in FIGS. 3A to 3D, regardless of presence or absence of HCV, total cholesterol, HDL-cholesterol, LDL-cholesterol, and triglycerides were all present at higher levels in the human serum than in the mouse serum. In addition, the cholesterol level decreased in the human serum in the presence of HCV, whereas it was not possible to identify changes in cholesterol level in the mouse serum even in the presence of HCV.

From the above results, it can be seen that an HCV envelope protein is well masked by lipids or lipoproteins in human blood because lipids adhere better to the envelope, which is HCV's surface, in human serum than in mouse (non-host) serum.

### [Example 4] Identification of stability of E2 surface antigen and genome of HCV, affected by bile acid

From the results of the above examples, based on the fact that human serum and mouse serum have different bile acid compositions, it was identified how HCV was affected by a bile acid.

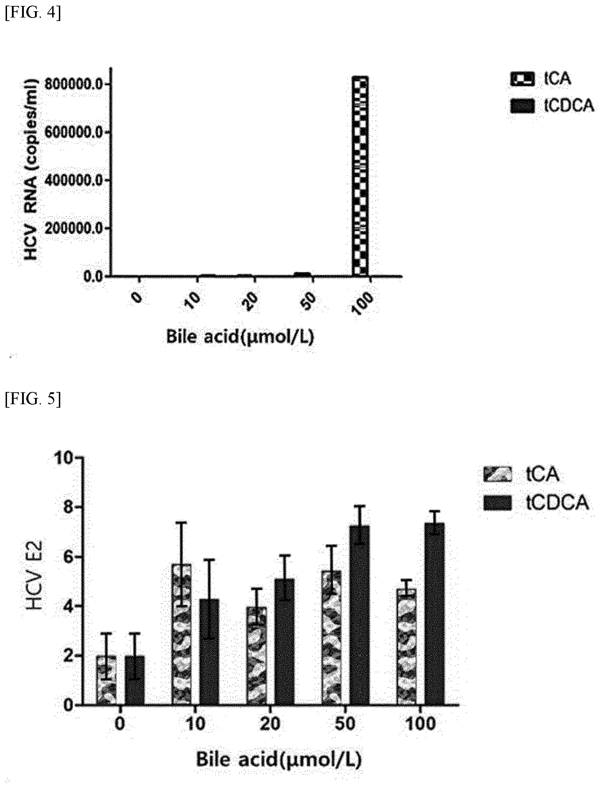

In the same manner as in Example 1, the HCV isolated in the preparation example was added to Dulbecco's Modified Eagle's Medium (DMEM), which is a cell culture medium, treatment with taurocholate (tCA) or taurochenodeoxycholate (tCDCA), which is a bile acid, at 0 to 100 µmol/L was performed, and culture was performed for 24 hours. Then, HCV RNA and HCV E2 antigen were detected in the same manner as in Example 1, and the results are illustrated in FIGS. 4 and 5.

As illustrated in FIG. 4, the HCV RNA was detected at a remarkably high level in a case where treatment with the bile acid tCA at 100 µmol/L was performed, as compared with a case where tCA was used at the other concentrations and a case where the other bile acid tCDCA was used.

As illustrated in FIG. 5, it was identified that with respect to the bile acids treated, the HCV E2 antigen increased in a dose-dependent manner; and that higher HCV surface antigenicity was observed in a case where treatment with tCDCA was performed, than in a case where treatment with tCA was performed.

From the above results, it can be seen that in a case where HCV is treated with a bile acid or a bile acid derivative, lipoproteins or the like, which mask the HCV's envelope, are removed so that HCV E2 surface antigen is exposed and can be detected, thereby achieving diagnosis of HCV infection in a more efficient manner.

### [Example 5] Identification of stability of E2 surface antigen and genome of HCV, affected by pH

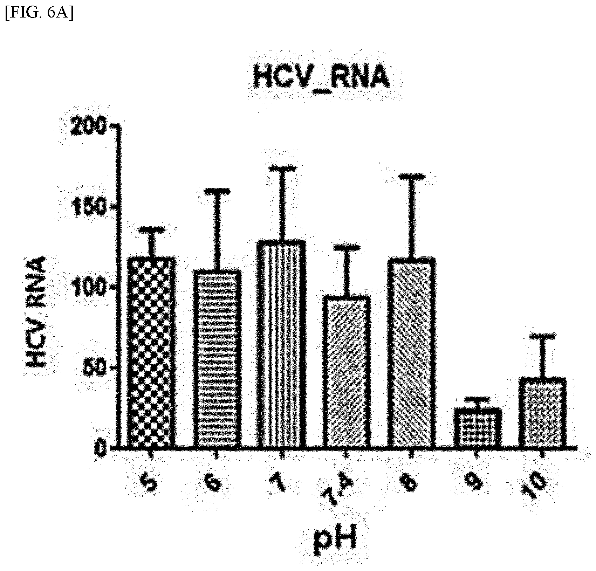

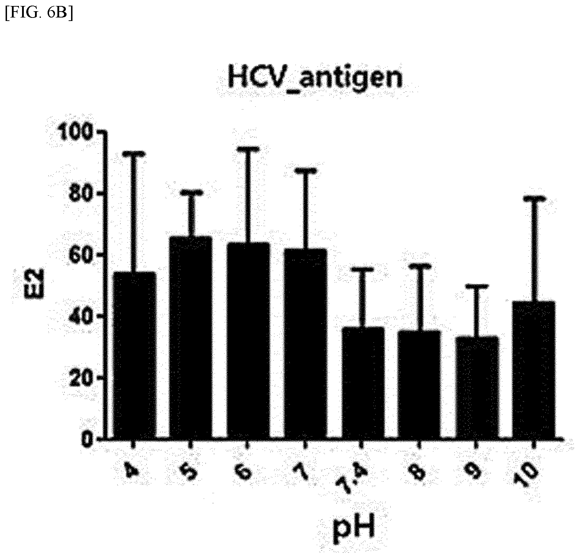

To DMEM, which is a cell culture medium containing no bile acid, was added the HCV isolated in the preparation example in a volume of 400 µl; and the pH of the DMEM was adjusted to pH 5, 6, 7.4, 8, 9, and 10 using HCl or NaOH. Then, culture was performed for 24 hours. Then, HCV RNA and HCV E2 antigen were detected in the same manner as in Example 1, and the results are illustrated in FIGS. 6A and 6B.

As illustrated in FIGS. 6A and 6B, the HCV RNA was detected at a higher level in a range of pH 5 to 6 (slightly acidic) and pH 7 than in pH 9 to 10 (basic). In addition, the HCV E2 antigen was detected at a higher level in a range of pH 5 to pH 7 than the basic pHs.

From the above results, it can be seen that in a case where HCV is exposed to an environment of pH 5 to pH 7, which is in a slightly acidic to neutral pH range, HCV surface antigen and RNA are exposed at high levels, and this can further facilitate detection of HCV.

Although specific parts of the present invention have been described in detail, it will be apparent to those skilled in the art that these specific descriptions are provided only for preferred embodiments and the scope of the present invention is not limited thereby. Therefore, the substantial scope of the present invention should be defined by the appended claims and equivalents thereof.

### Industrial applicability

The method for detecting a virus of the present invention allows a surface antigen, which is a lipid bilayer envelope protein, to be effectively exposed from lipoproteins that mask all or part of the envelope protein, thereby revealing the surface antigen that is masked by the lipoproteins, so that a probe such as an antibody can be used to detect the virus with high sensitivity within a short time.

## Claims

1. A method for pretreating a sample for virus detection, comprising steps of:
isolating an enveloped virus including a lipid bilayer envelope, from a biological sample isolated from a target individual; and
treating the enveloped virus with any one or more selected from the group consisting of a bile acid, a bile acid derivative, and a pH adjusting agent.

2. The method according to claim 1, wherein the treating step is carried out such that an envelope protein is exposed from the lipid bilayer.

3. The method according to claim 2, wherein the envelope protein is E1 or E2 protein, which is a surface antigen, of the enveloped virus.

4. The method according to claim 1, wherein the enveloped virus including a lipid bilayer envelope is a hepatitis virus.

5. The method according to claim 4, wherein the hepatitis virus is hepatitis C virus (HCV).

6. The method according to claim 1, wherein the bile acid derivative is any one or more selected from the group consisting of chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid.

7. The method according to claim 1, wherein the bile acid or the bile acid derivative is used at a concentration of 10 to 100 µmol/L.

8. The method according to claim 1, wherein the pH adjusting agent is used to adjust the pH of a culture environment for the enveloped virus to pH 4 to pH 7.

9. A composition for pretreating a sample for enveloped virus detection, comprising as an active ingredient:
any one or more selected from the group consisting of a bile acid, a bile acid derivative, and a pH adjusting agent.

10. The composition according to claim 9, wherein the enveloped virus includes a lipid bilayer envelope.

11. The composition according to claim 9, wherein the bile acid derivative is any one or more selected from the group consisting of chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid.

12. The composition according to claim 9, wherein the pH adjusting agent is used to adjust the pH of a culture environment for the enveloped virus to pH 4 to pH 7.

13. A method for detecting an enveloped virus, comprising steps of:
isolating an enveloped virus including a lipid bilayer envelope, from a biological sample isolated from a target individual;
treating the enveloped virus with any one or more selected from the group consisting of a bile acid, a bile acid derivative, and a pH adjusting agent; and
adding thereto an antibody specific for the virus.

14. The method according to claim 13, wherein the treating step is carried out such that an envelope protein is exposed from the lipid bilayer.

15. The method according to claim 13, wherein the antibody is specific for an envelope protein of the enveloped virus, and the envelope protein is E1 or E2 protein that is a surface antigen.

16. The method according to claim 13, wherein the enveloped virus including a lipid bilayer envelope is a hepatitis virus.

17. The method according to claim 16, wherein the hepatitis virus is hepatitis C virus (HCV).

18. The method according to claim 13, wherein the bile acid derivative is any one or more selected from the group consisting of chenodeoxycholic acid, deoxycholic acid, lithocholic acid, taurocholic acid, glycocholic acid, taurochenodeoxycholic acid, glycochenodeoxycholic acid, glycodeoxycholic acid, taurodeoxycholic acid, glycolithocholic acid, taurolithocholic acid, and muricholic acid.
